# EUROPEAN PATENT SPECIFICATION

(11) **EP 4 230 237 B1**
(45) Date of publication and mention of the grant of the patent: **24.09.2025**
(21) Application number: 20957656.0
(22) Date of filing: 14.10.2020
(51) Int. Cl.: A61M 1/36

(54) **PHOTODYNAMIC THERAPY DEVICE COMPRISING COOLING WATER CIRCULATION UNIT FOR BLOOD**
PHOTODYNAMISCHE THERAPIEVORRICHTUNG MIT KÜHLWASSERZIRKULATIONSEINHEIT FÜR BLUT
DISPOSITIF DE THÉRAPIE PHOTODYNAMIQUE COMPRENANT UNE UNITÉ DE CIRCULATION D'EAU DE REFROIDISSEMENT POUR LE SANG

(43) Date of publication of application: 23.08.2023
(73) Proprietor: Otsuka Electronics Co., Ltd., Hirakata-shi, Osaka 573-1132 (JP)
(72) Inventor: HAMAO, Tamotsu, Hirakata-shi, Osaka 573-1132 (JP); TANAKA, Akira, Hirakata-shi, Osaka 573-1132 (JP); KAJIWARA, Shinpei, Hirakata-shi, Osaka 573-1132 (JP)
(74) Representative: Hoffmann Eitle
(86) International application number: PCT/JP2020/038764
(87) International publication number: WO 2022/079827

(56) References cited:
- WO-A1-2020/174589
- JP-A- 2005 518 837
- JP-A- 2013 517 093
- US-A- 5 290 221
- US-A1- 2003 163 183
- US-A1- 2009 156 976
- US-A1- 2011 178 580
- US-A1- 2019 209 718
- US-A1- 2019 374 701

## Description

### TECHNICAL FIELD

The present disclosure relates to a photodynamic therapy device including a cooling water circulation unit for blood.

### BACKGROUND ART

In photodynamic therapy (PDT), blood having absorbed a photoreactive agent having a characteristic light absorption band is once taken out of a patient's body and irradiated with a light beam corresponding to the characteristic light absorption band, thereby destroying or affecting undesirable components in the blood. For example, an LED is used as a light source of a light beam which is irradiation light.

By the way, when the blood taken out of the patient's body is irradiated with the light beam by the LED at the time of the PDT treatment described above, the blood generates heat and the temperature rises. An increase in the temperature of the blood may place a burden on the patient's body to which the blood is returned. Furthermore, an increase in temperature adversely affects the blood itself.

### PRIOR ART DOCUMENT

### PATENT DOCUMENT

Patent Document 1: WO 2017/164202 A

WO 2020/174589 A1 discloses a photodynamic therapy device that efficiently radiates light at the blood of a patient. This photodynamic therapy device includes: a cartridge that includes a winding core and a tube that is wound around the winding core; a housing that houses the cartridge; and a light source that is arranged inside the housing and radiates light at the tube. In cross-sections taken perpendicular to the extension direction thereof, the tube that is wound around the winding core has a first dimension in a first direction and, in a second direction that is perpendicular to the first direction, a second dimension that is smaller than the first dimension. The second direction is directed to the outside of the winding core.

US 2011/178580 A1 discloses enhanced methods and improved devices to eliminate, reduce, destroy and/or inhibit undesired body fluid species, such as pathogen microbes and deterio-rated or malignant cells in complex environments like blood, serum and other body fluids. In preferred embodiments, present invention provides an antimicrobial PDT treatment that effectively inactivates, reduces and/or destroys both Gram (-) and Gram (+) bacteria in complex body fluids. Methods to enhance antimicrobial PDT activity includes the steps of administering a photosensitizer to bacteria-contaminated fluid, after a dwell time guiding bacteria-contaminated fluid with photosensitizer through a channel, emitting radiation preferably in an intermittent manner, and restoring treated body fluids to corresponding body regions. Electro-magnetic radiation is preferably delivered intermittently with pulse width based on treatment parameters. Preferred device embodiments comprise guiding channels and at least one electromagnetic radiation source, arranged separately or in sequence. Preferably, laser device or LED-panels are used to deliver electromagnetic radiation to activate the photosensi-tizer. When used with preferred photosensitizer composition based on Safranin O, preferred laser radiation wavelength is in the range of 500-580 nm. Additionally, present invention diminishes adverse host's inflammatory responses by neutral-izing the biological activity of pathogenic microorganism fragments and reducing and/or removing pathogenic micro-organism fragments responsible for it.

US 2003/163183 A1 discloses a temperature control system which provides for the optimal management of patient temperature during a surgical procedure, such as those which require the patient to on bypass. The system employs a plurality of controllers as well as a plurality of temperature control means in order to provide optimal temperature control. In one configuration of the invention, controllers for each of the heat exchange devices may be interconnected using a data link. The connection may provide for a master/slave relationship wherein temperature sensors included in each system are employable as temperature feedback for initiating temperature changes. This device may be configured such that it operates in conjunction with another device or provides stand alone temperature control.

US 2009/156976 A1 discloses methods and devices provided for the extracorporeal ablation of target cells circulating in the blood of an organism. Exogenous material introduced into the blood preferentially associates with target cells (e.g. cancer cells, bacteria, viruses) in the blood. An extracorporeal continuous flow pathway accesses the patient's blood to apply an external energy source to the blood at an ex vivo ablation device in a portion of the extracorporeal continuous flow pathway. The exogenous material interacts with the applied energy so as to result in the damage or death of the target cells. The blood is then returned to the body in a continuous-flow pattern. By applying the energy while the blood is in the ex vivo ablation device, shielding of the target cells by the body is reduced and detrimental effects on the organs and tissues of the body are avoided or mitigated.

US 5 290 221 A discloses systems for treating a fluid carrying a contaminant to which a photoactive material has been bound and which include a treatment device that defines a relatively narrow, accurately shaped flow path. These systems envelop the path with a radiation chamber that directs radiation from one or more sources into the fluid.

US 2019/374701 A1 discloses a microfluidic photoreactor for treating excess bilirubin in blood, having: a microfluidic channel module; an illumination module comprising one or more illumination sources disposed about the microfluidic channel module and configured to illuminate blood passing through at least one microfluidic channel of the microfluidic channel module; and a heat exchanger module coupled to the at least one microfluidic channel module, wherein the heat exchanger module is configured to extract heat from the at least one microfluidic channel. A system including a micro fluidic photoreactor and a method of treating excess bilirubin in blood.

### SUMMARY OF THE INVENTION

### PROBLEMS TO BE SOLVED BY THE INVENTION

The present disclosure provides a photodynamic therapy device that suppresses an increase in blood temperature in PDT.

### MEANS FOR SOLVING THE PROBLEMS

The photodynamic therapy device of the present disclosure is a photodynamic therapy device that irradiates light from a light source onto blood that has been taken out of a patient's body and is flowing in a blood tube, the blood having absorbed a photoreactive agent, to destroy an undesirable component in the blood or to affect the component. A photodynamic therapy device according to the present invention is defined by claim 1.

Dependent claims relate to preferred embodiments.

### EFFECTS OF THE INVENTION

In the photodynamic therapy device of the present disclosure, a temperature rise caused by the heat generation of the blood due to the absorption of the irradiation light is suppressed.

### BRIEF DESCRIPTION OF THE DRAWINGS

FIG. 1A is a front view of a photodynamic therapy device according to an embodiment of the present disclosure.
FIG. 1B is a rear perspective view of the photodynamic therapy device according to the embodiment.
FIG. 2A is a perspective view of a circuit holder, a circuit cooling block, and an irradiation unit in the photodynamic therapy device according to the embodiment. An arrangement of the irradiation unit, the circuit holder, and the circuit cooling block in the drawing is at the time of monitoring an amount of light.
FIG. 2B is a perspective view of the circuit holder, the circuit cooling block, and the irradiation unit in the photodynamic therapy device according to the embodiment. An arrangement of the irradiation unit, the circuit holder, and the circuit cooling block in the drawing is at the time of treatment.
FIG. 3A is a substantially front perspective view of the circuit holder and the circuit cooling block in the photodynamic therapy device according to the embodiment.
FIG. 3B is a perspective view of the circuit holder and the circuit cooling block in the photodynamic therapy device according to the embodiment.
FIG. 4A is a perspective view of the circuit holder in the photodynamic therapy device according to the embodiment.
FIG. 4B is a perspective view from a bottom surface of the circuit holder in the photodynamic therapy device according to the embodiment.
FIG. 4C is an exploded perspective view of the circuit holder in the photodynamic therapy device according to the embodiment.
FIG. 5 is a perspective view of the photodynamic therapy device according to the embodiment in a state where a blood tube is wound around in the circuit holder.
FIG. 6A is a perspective view of the circuit cooling block in the photodynamic therapy device according to the embodiment.
FIG. 6B is a partial cross-sectional view taken along a horizontal plane including line IB-IB in FIG. 6A in the circuit cooling block.
FIG. 7 is a perspective view of a circuit cooling sub-block constituting the circuit cooling block in the photodynamic therapy device according to the embodiment, and illustrates a tube through which cooling water flows.
FIG. 8 is a schematic diagram of a cooling water circulation unit connected to the photodynamic therapy device according to the embodiment, and the cooling water circulation unit in the drawing is in a state at the time of preparation for treatment.
FIG. 9 is a schematic diagram of the cooling water circulation unit connected to the photodynamic therapy device according to the embodiment, and the cooling water circulation unit in the drawing is in a state at the time of treatment.

### MODES FOR CARRYING OUT THE INVENTION

In the following, an embodiment will be described in detail with reference to the drawings as appropriate. However, unnecessarily detailed descriptions will be omitted in some cases. For example, detailed descriptions of already well-known matters and repetition of descriptions of substantially the same configuration will be omitted in some cases. This is to prevent the following description from being unnecessary redundant and to facilitate those skilled in the art to understand the present disclosure.

Note that the inventors provide the accompanying drawings and the following description in order for those skilled in the art to fully understand the present disclosure, and does not intend to limit the subject matter described in the claims by the accompanying drawings and the following description.

### 1. [Background to present disclosure]

Photodynamic therapy (PDT) is a treatment method in which a photosensitizer or a precursor thereof is administered, the photosensitizer or the precursor thereof is accumulated in an affected area such as a tumor tissue, a new blood vessel, or a skin surface, a light beam corresponding to an absorption band wavelength of the photosensitizer is irradiated as excitation light, and a cytocidal effect of active oxygen species including singlet oxygen generated by the excitation is utilized.

Taking an example in which a patient is a hematologic cancer patient and has tumor cells, in photodynamic therapy, first 5-aminolevulinic acid (5-ALA) having oral absorbability is administered to the patient. Then, in the process of biosynthesis of heme in intracellular mitochondria, aminolevulinic acid is metabolized to protoporphyrin IX (PpIX) which is a photosensitizer. Since protoporphyrin IX has a property of accumulating in mitochondria in a tumor cell specific manner, protoporphyrin IX accumulates in tumor cells of a patient.

Subsequently, in the photodynamic therapy, a patient's circulatory organ is connected to an irradiation device for the photodynamic therapy via a blood circuit for flowing blood. The blood of the patient contains tumor cells in which the protoporphyrin IX is accumulated, and the blood flows into the irradiation device through the blood circuit by the action of a circulation pump connected to the blood circuit for flowing blood. In the irradiation device, when light in a wavelength range (for example, in the vicinity of 410 nm and in the vicinity of 500 to 650 nm) where protoporphyrin IX can absorb is irradiated, the protoporphyrin IX contained in the blood becomes in an excited singlet state. The protoporphyrin IX returns from the excited singlet state to the ground state through an excited triplet state. Oxygen that has absorbed the energy at that time becomes singlet oxygen, and can destroy or affect tumor cells in the blood. The blood that has been irradiated with light is returned to the patient's circulatory organ by the action of the circulation pump via the blood circuit for flowing blood.

The blood circuit is connected to a translucent blood tube formed of a predetermined resin in the irradiation device. The blood tube through which blood flows inside is irradiated with a light beam corresponding to an absorption band wavelength of protoporphyrin IX, which is a photosensitive substance, by an LED, which is a light source, for example. At this time, the inventors have noticed that the blood may generate heat due to absorption of the irradiation light, and a temperature of the blood may rise. In particular, the illuminance of the light source must be correspondingly increased in order to obtain a sufficient effect for the photodynamic therapy at a treatment time (for example, 3 hours) that does not significantly affect the patient's physical strength. In that case, according to trial calculation and trial by the inventors, the temperature of the blood may reach 60°C. An increase in the temperature of the blood imposes a burden of a high temperature on the patient's body to which the blood is returned. Moreover, the increase in temperature adversely affects the blood itself.

Note that the inventors have found that it is desirable that the temperature of the blood that can be increased by the irradiation device is 40°C or lower at most by a large number of trials and trial calculations.

The present disclosure overcomes these problems, and provides a photodynamic therapy device that suppresses a temperature rise of blood due to irradiation light in the photodynamic therapy.

### 2. [Embodiment]

Hereinafter, preferred embodiments of the present disclosure will be described with reference to the accompanying drawings.

### 2.1. [Configuration of photodynamic therapy device]

First, a configuration of a photodynamic therapy device according to an embodiment will be described.

**2.1.1.** [Schematic configuration of photodynamic therapy device]

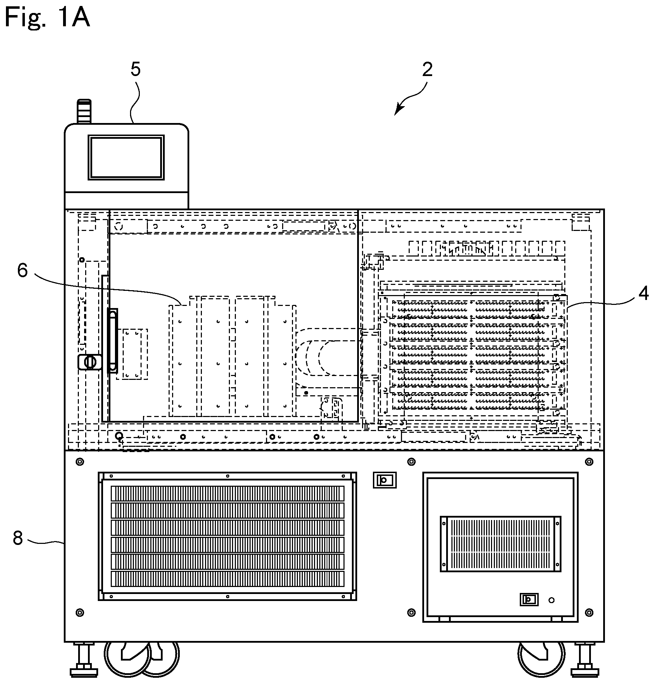

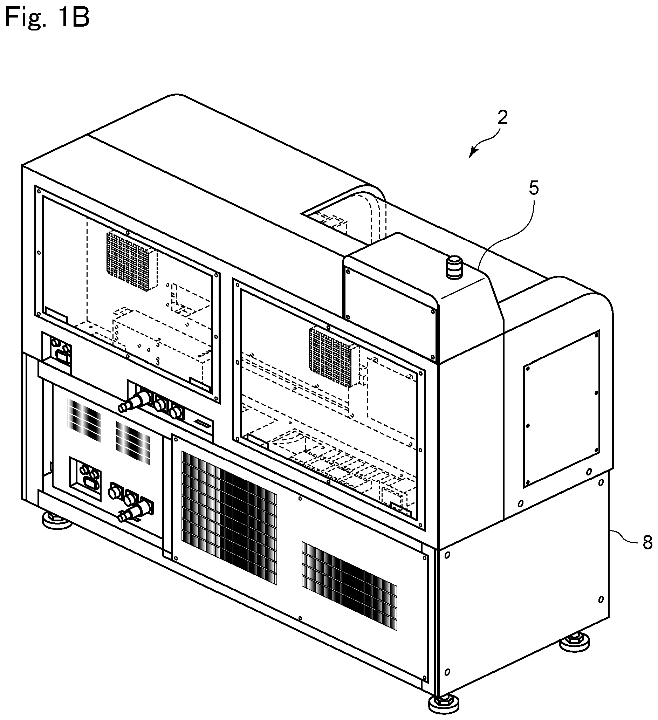

FIG. 1A is a front view of a photodynamic therapy device 2 according to the embodiment. FIG. 1B is a rear perspective view of the photodynamic therapy device 2 according to the same embodiment.

As illustrated in FIG. 1A, the photodynamic therapy device 2 includes a circuit cooling block 6 and an irradiation unit 4.

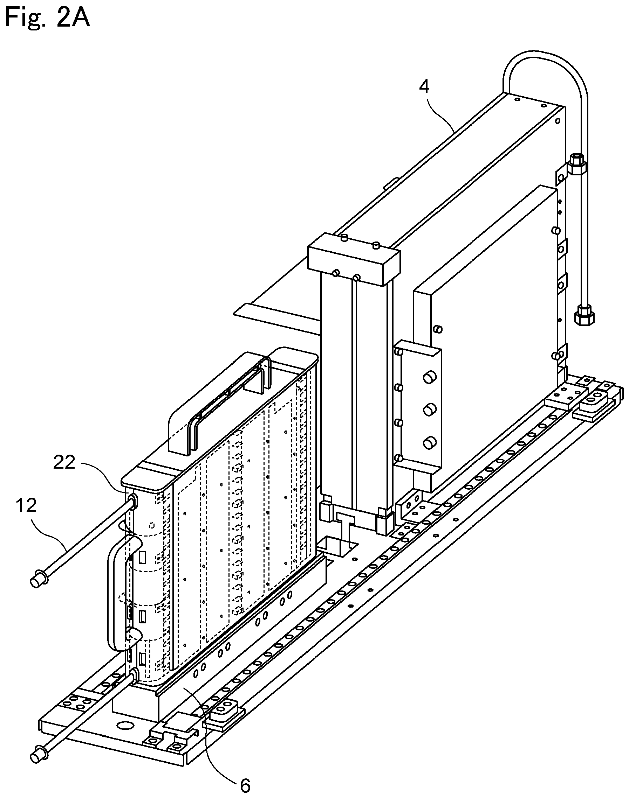

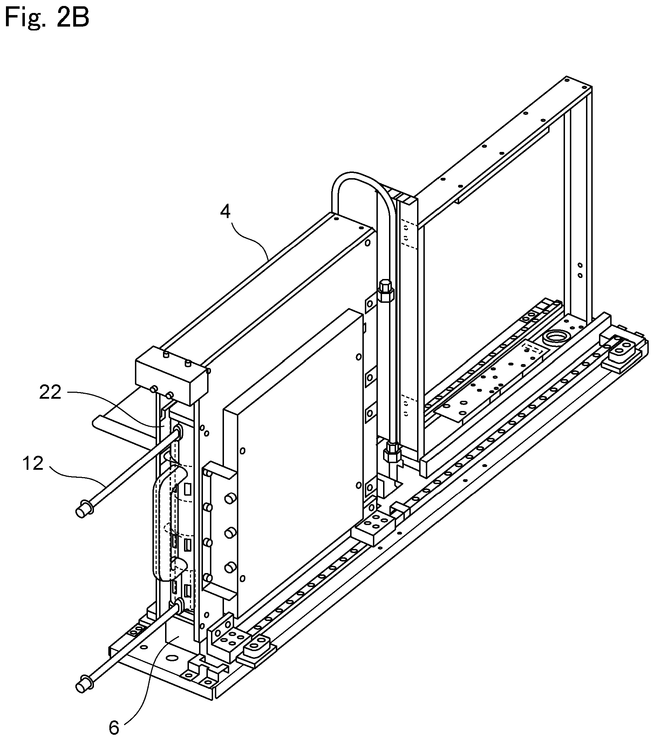

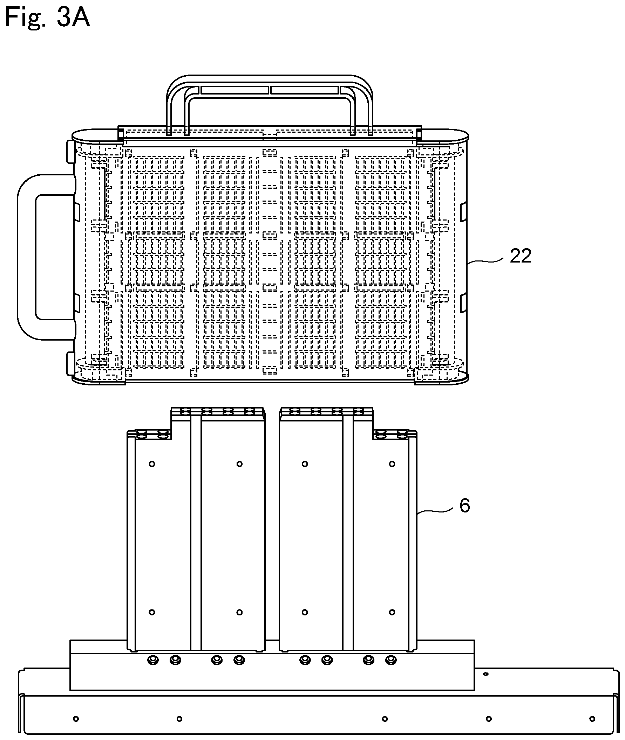

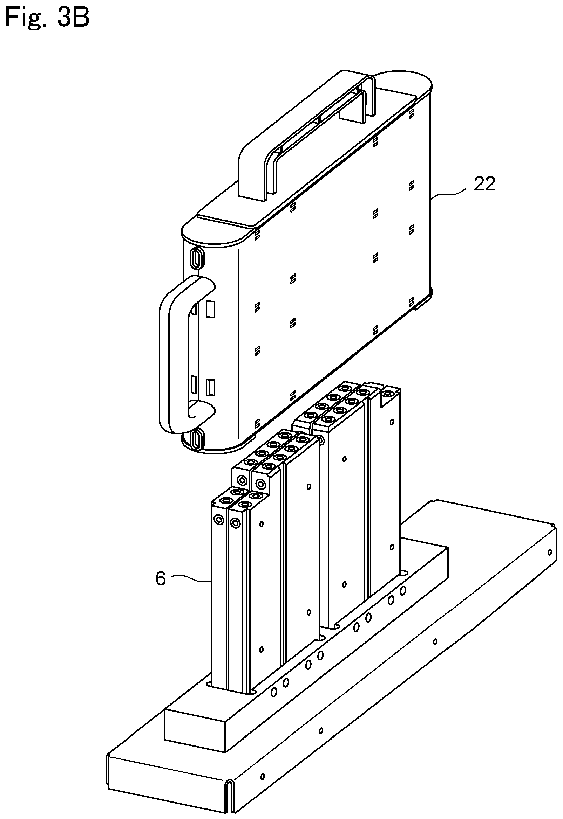

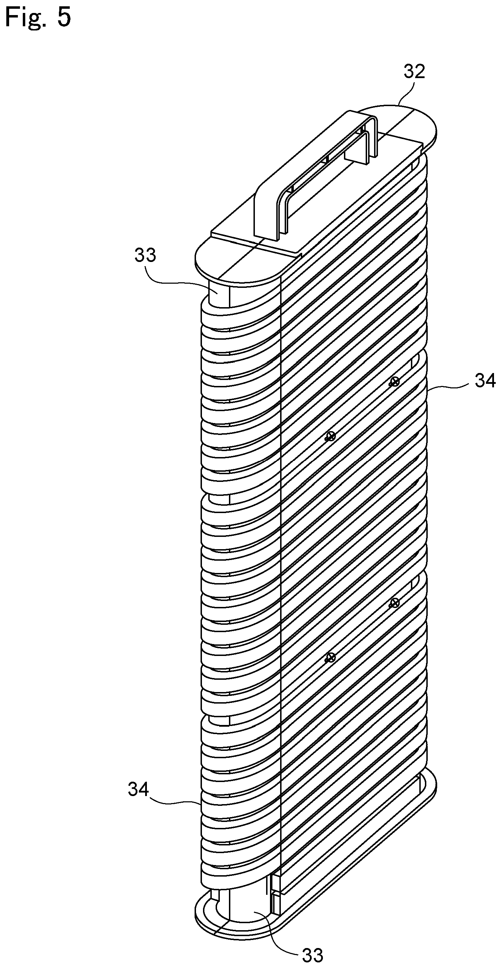

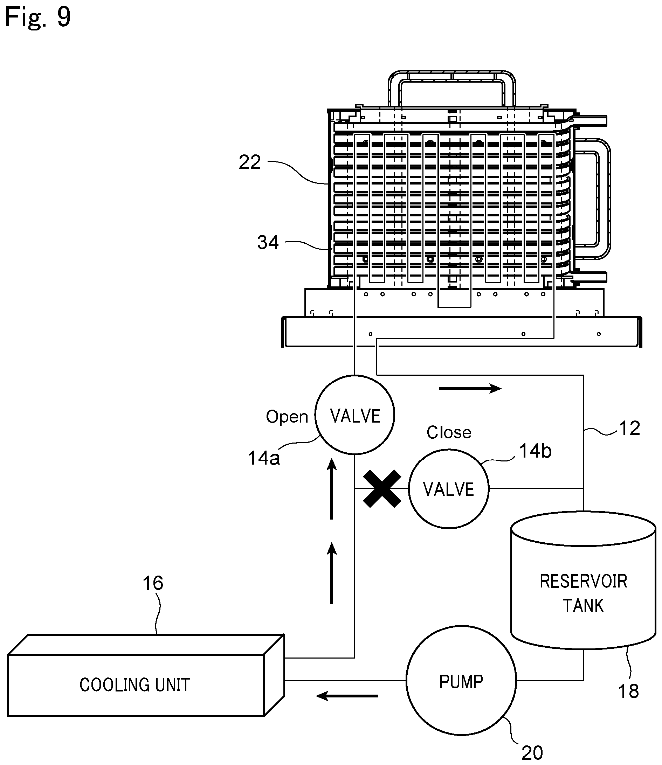

The circuit cooling block 6 is formed of a predetermined material, and as will be described later, is inserted into a circuit holder 22 (See FIGS. 3A, 3B, etc.) including a blood tube 34 (See FIGS. 5 and 9.) connected to a blood circuit for flowing blood (, that is, forming part of the blood circuit), and comes into contact with an inner surface of the circuit holder 22 to cool the blood tube 34 and the blood. The material forming the circuit cooling block 6 is desirably a metal having high thermal conductivity, for example, aluminum. The irradiation unit 4 irradiates the blood tube and the blood in the circuit holder 22 with a light beam corresponding to a characteristic light absorption band. The irradiation unit 4 includes, inside the irradiation unit 4, a light source (not illustrated) including a large number of light emitting elements (for example, LEDs) and a light detection unit (not illustrated) including light detection elements. A large number of the light emitting elements constituting the light source are arranged, for example, in a matrix inside a pair of left and right wider side surface portions (See FIGS. 2A and 2B.) facing each other of the irradiation unit 4. The light detection unit is disposed, for example, inside an upper surface and inside a bottom surface of the irradiation unit 4.

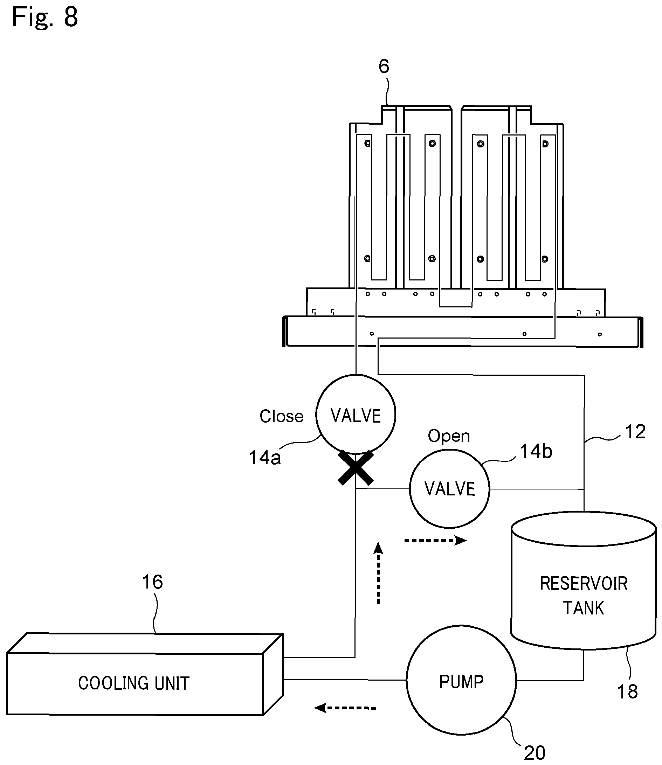

The photodynamic therapy device 2 illustrated in FIG. 1A is further provided with a lower casing 8. The lower casing 8 houses a cooling unit 16, a first valve 14a and a second valve 14b, a reservoir tank 18, and a pump 20 that constitute a cooling water circulation unit described later with reference to FIGS. 8 and 9.

Furthermore, in the photodynamic therapy device 2 illustrated in FIGS. 1A and 1B, an opening/closing operation of the valves to be described later and the like are performed through an operation on an operation unit 5 provided in an upper portion.

### 2.1.2. [Configuration of circuit holder, circuit cooling block, and irradiation unit]

FIG. 2A is a perspective view of the circuit holder 22, the circuit cooling block 6, and the irradiation unit 4 in the photodynamic therapy device 2 according to the embodiment. As also illustrated in FIG. 2A, the circuit cooling block 6 is inserted inside the circuit holder 22. The irradiation unit 4 is configured to be relatively movable with respect to the circuit holder 22 and the circuit cooling block 6. Note that, in the embodiment illustrated in FIGS. 2A and 2B, the irradiation unit 4 can move with respect to the fixed circuit cooling block 6. In a state where the circuit holder 22 and the circuit cooling block 6 are separated from the irradiation unit 4, the irradiation unit 4 monitors a light amount using the light source and the light detection unit.

FIG. 2B is also a perspective view of the circuit holder 22, the circuit cooling block 6, and the irradiation unit 4. The circuit holder 22 and the circuit cooling block 6 are accommodated inside the irradiation unit 4 by movement of the irradiation unit 4 relative to the circuit holder 22 and the circuit cooling block 6. At this time, the pair of left and right light sources inside the irradiation unit 4 are arranged to face a pair of side surfaces of the circuit holder 22. The pair of left and right light sources of the irradiation unit 4 is arranged to face the pair of side surfaces of the circuit holder 22, and treatment by light irradiation of the irradiation unit 4 is performed.

### 2.1.3. [Configuration of circuit holder and circuit cooling block]

FIG. 3A is a substantially front perspective view of the circuit holder 22 and the circuit cooling block 6 in a separated state in the photodynamic therapy device 2 according to the embodiment. FIG. 3B is also a perspective view of the circuit holder 22 and the circuit cooling block 6 in a separated state in the photodynamic therapy device 2 according to the embodiment.

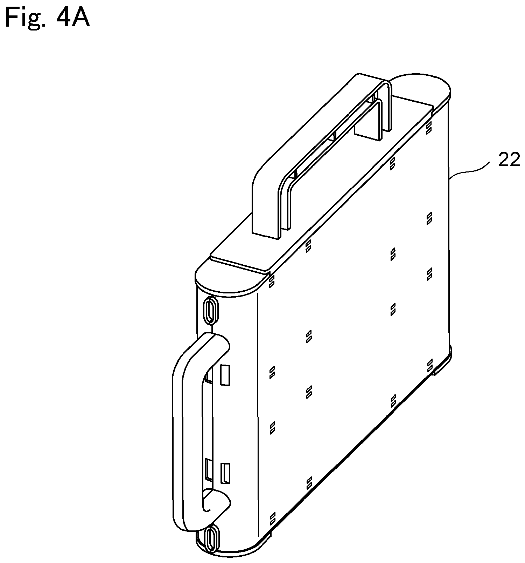

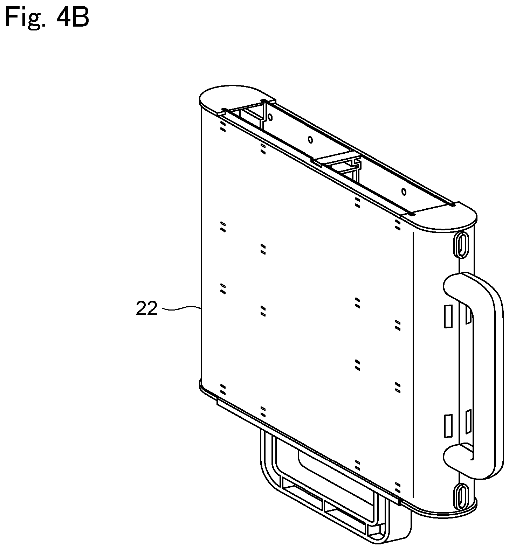

FIG. 4A is a perspective view of the circuit holder 22 in the photodynamic therapy device 2 according to the embodiment, and FIG. 4B is a perspective view from a bottom surface of the circuit holder 22 in the photodynamic therapy device 2 according to the embodiment. In particular, as illustrated in FIG. 4B, the inside of the circuit holder 22 is hollow, and the circuit cooling block 6 is inserted into this hollow portion and comes into contact with the inner surface of the circuit holder 22.

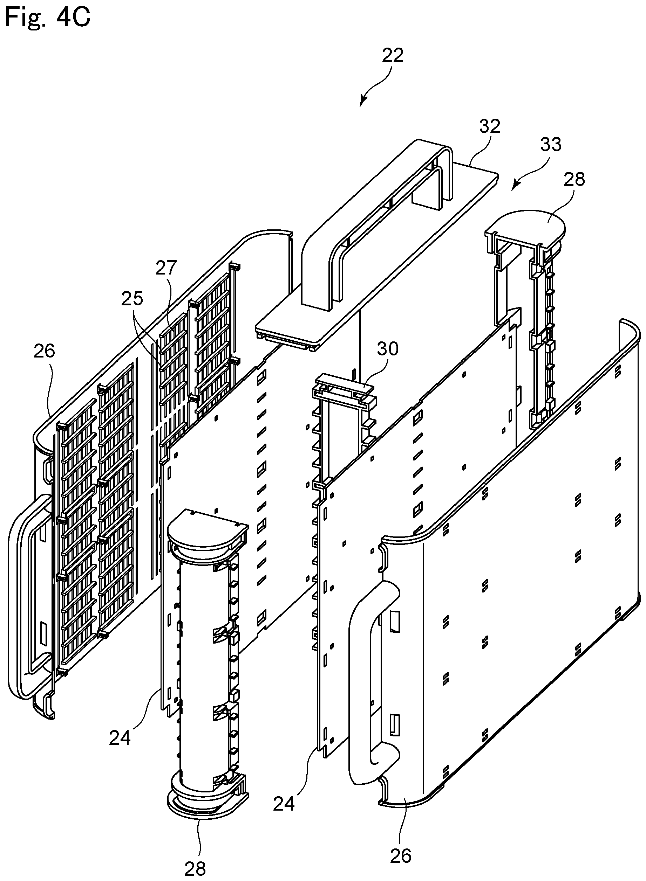

FIG. 4C is an exploded perspective view of the circuit holder 22 in the photodynamic therapy device 2 according to the embodiment. As illustrated in FIG. 4C, the circuit holder 22 has a combination structure of four types of molded products and two aluminum sheet metals 24. Here, the four types of molded products are a circuit holder center portion 30, two circuit holder end portions 28, two circuit holder side surface portions 26, and a circuit holder upper surface portion 32.

Note that in the exploded view of the circuit holder 22 illustrated in FIG. 4C, the blood tube 34 connected to the blood circuit for flowing blood (, that is, forming part of the blood circuit) is omitted. In the circuit holder 22, the blood tube 34 is wound around a winding core portion 33 including the two aluminum sheet metals 24, the circuit holder center portion 30, the two circuit holder end portions 28, and the circuit holder upper surface portion 32 (See FIG. 5.). A guide for winding the blood tube 34 is appropriately provided on the aluminum sheet metals 24, the circuit holder center portion 30, and the circuit holder end portions 28 so that the blood tube 34 is regularly wound around the winding core portion 33.

Each of the two circuit holder side surface portions 26 is formed of a transparent resin thin plate, for example, a polycarbonate thin plate in order to cause the irradiation light from the light source of the irradiation unit 4 to reach the blood tube 34 and the blood flowing therein. As illustrated by the circuit holder side surface portion 26 in FIG. 4C, a lateral guide 25 for winding the blood tube 34 is also appropriately provided inside the circuit holder side surface portion 26.

Furthermore, a large number of thin ribs 27 in a longitudinal direction are also provided inside the circuit holder side surface portion 26. The large number of ribs 27 in the longitudinal direction improve the adhesion of the blood tube 34 to the aluminum sheet metal 24.

Most of the blood tube 34 wound around the winding core portion 33 of the circuit holder 22 is sandwiched between the circuit holder side surface portion 26 that transmits the irradiation light from the light source of the irradiation unit 4 and the aluminum sheet metal 24 cooled by being in direct contact with the circuit cooling block 6. FIG. 5 is a perspective view of a state where the blood tube 34 is wound around the winding core portion 33 in the circuit holder 22 in the photodynamic therapy device 2 according to the embodiment.

Note that a transparent thin film cover may be provided between the circuit holder side surface portion 26 and the blood tube 34, or the circuit holder side surface portion 26 and the blood tube 34 may be in direct contact with each other without sandwiching anything therebetween. A cover made of a transparent thin film may also be provided between the aluminum sheet metal 24 and the blood tube 34, or the aluminum sheet metal 24 and the blood tube 34 may be in direct contact with each other without sandwiching anything therebetween.

### 2.1.4. [Configuration of circuit cooling block]

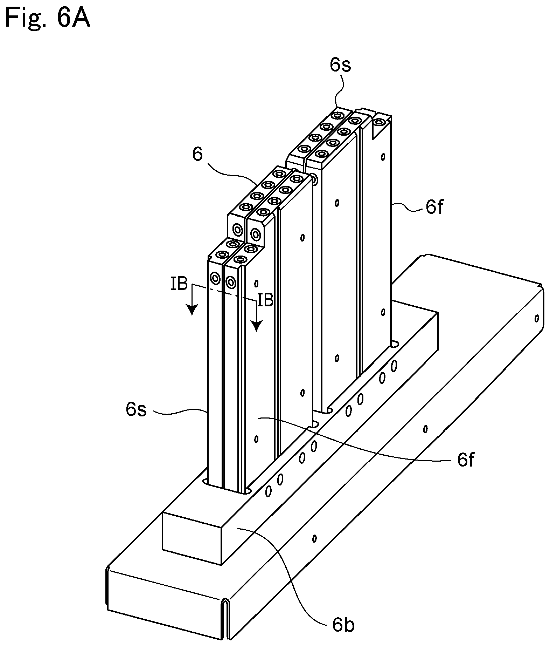

FIG. 6A is a perspective view of the circuit cooling block 6 in the photodynamic therapy device 2 according to the embodiment. The circuit cooling block 6 includes four circuit cooling sub-blocks (6f, 6s) and a pedestal portion 6b provided with the circuit cooling sub-blocks (6f, 6s). The four circuit cooling sub-blocks (6f, 6s) include two fixed circuit cooling sub-blocks 6f and two sliding circuit cooling sub-blocks 6s. The "fixed" and the "sliding" will be described later. Note that the four circuit cooling sub-blocks (6f, 6s) may have substantially the same structure. Further, the circuit cooling sub-blocks (6f, 6s) are desirably made of aluminum. Furthermore, the number of circuit cooling sub-blocks (6f, 6s) may be more than four.

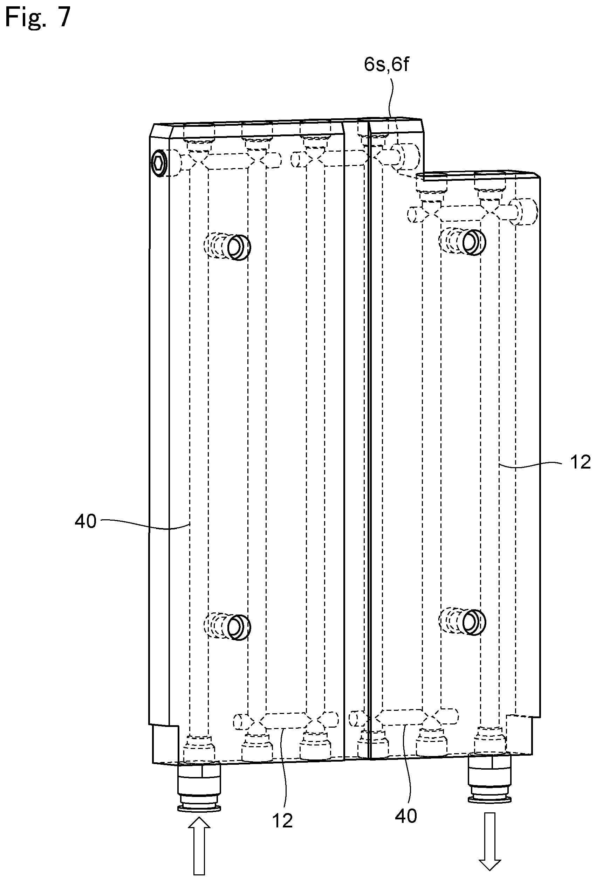

FIG. 7 is a perspective view of the circuit cooling sub-blocks (6f, 6s) constituting the circuit cooling block 6 in the photodynamic therapy device 2 according to the embodiment, and illustrates tubes 40 through which cooling water flows. The cooling water flows in from the pedestal portion 6b, flows in the tubes 40 vertically and horizontally provided inside the circuit cooling sub-blocks (6f, 6s), and flows out to the pedestal portion 6b. The tubes 40 inside the circuit cooling sub-blocks (6f, 6s) and the pedestal portion 6b form a part of a water flow path 12 (See FIGS. 8 and 9.) of a cooling water circulation unit to be described later.

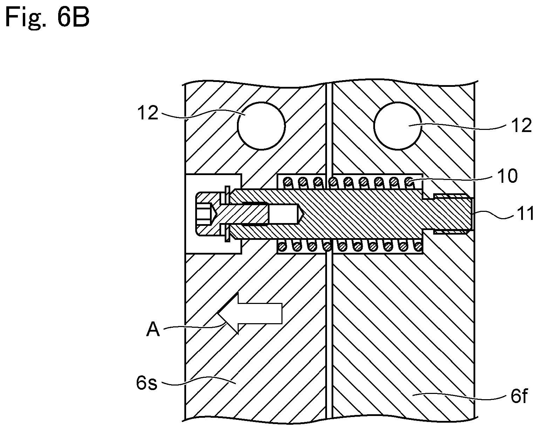

FIG. 6B is a partial cross-sectional view taken along a horizontal plane including line IB-IB in FIG. 6A in the circuit cooling block 6. One of the circuit cooling sub-blocks constituting the circuit cooling block 6 is of a fixed type (fixed circuit cooling sub-block 6f). The other of the circuit cooling sub-blocks is of a sliding type (sliding circuit cooling sub-block 6s). As illustrated in FIG. 6B, an elastic body 10 is sandwiched between the fixed circuit cooling sub-block 6f and the sliding circuit cooling sub-block 6s provided to face each other by being engaged with respective recesses. In the circuit cooling block 6 illustrated in FIG. 6B, a spring is used as an example of the elastic body 10.

A shaft 11 passes through the elastic body 10. This shaft 11 is configured to be fixed relative to the sliding circuit cooling sub-block 6s and slidable relative to the fixed circuit cooling sub-block 6f. This allows the sliding circuit cooling sub-block 6s to slide with respect to the fixed circuit cooling sub-block 6f while accurately maintaining a parallel positional relationship between the fixed circuit cooling sub-block 6f and the sliding circuit cooling sub-block 6s. The shaft 11 may be configured to be fixed relative to the fixed circuit cooling sub-block 6f and slidable relative to the sliding circuit cooling sub-block 6s.

In the pair of fixed circuit cooling sub-blocks 6f and sliding circuit cooling sub-blocks 6s facing each other, a plurality of the elastic bodies 10, for example, four elastic bodies are provided.

As described above, by setting the plurality of elastic bodies 10 to be sandwiched, the sliding circuit cooling sub-block 6s is biased in a direction of an arrow A illustrated in FIG. 6B, that is, in an outward direction. Since the sliding circuit cooling sub-block 6s is biased outward by the elastic bodies 10, when the circuit cooling block 6 is inserted into the circuit holder 22, the circuit cooling sub-blocks (6s, 6f) come into stronger contact with the inner surface of the aluminum sheet metal 24 of the circuit holder 22.

That is, in the two circuit cooling sub-blocks (6s, 6f) facing each other, one is fixed, and the other is held in a state where a reaction force is applied in a facing direction by the elastic body mechanism. This is to improve the adhesion of the circuit cooling sub-blocks (6s, 6f) to the aluminum sheet metal 24 of the circuit holder 22. This enhances the cooling action of the circuit cooling block 6 on the aluminum sheet metal 24 of the circuit holder 22, the blood tube 34, and thus the blood in the blood tube 34.

That is, the temperature of the blood is transferred and heat-exchanged with the blood tube 34 → the aluminum sheet metal 24 → the circuit cooling block 6, so that the increase in the blood temperature is suppressed.

Note that the circuit cooling block 6 may be configured such that, in the two circuit cooling sub-blocks facing each other, both are the sliding circuit cooling sub-blocks 6s, and the sliding circuit cooling sub-blocks 6s are biased outward by the elastic mechanism.

### 2.1.5. [Configuration of cooling water circulation unit]

FIGS. 8 and 9 are schematic diagrams illustrating configurations of the circuit holder 22, which are accompanied by the blood tube 34 connected to the blood circuit for flowing blood (, that is, forming part of the blood circuit), the circuit cooling block 6, and the cooling water circulation unit. The cooling water circulation unit in FIG. 8 is in a state at the time of preparation for treatment. The cooling water circulation unit in FIG. 9 is in a state at the time of treatment.

As illustrated in FIGS. 8 and 9, the cooling water circulation unit includes a cooling unit 16, a first valve 14a and a second valve 14b, a reservoir tank 18, and a pump 20. The cooling unit 16, the first valve 14a, the circuit cooling block 6, the second valve 14b, the reservoir tank 18, and the pump 20 are connected by the water flow path 12 through which cooling water flows.

(In FIGS. 8 and 9, for the sake of convenience of drawing and presentation, the water flow path is illustrated in the foreground, and however,) the water flow path 12 is also provided vertically and horizontally inside the circuit cooling block 6 (See FIG. 7.). The first valve 14a is provided on an upstream side of the circuit cooling block 6 between the cooling unit 16 and the circuit cooling block 6. The second valve 14b is provided on an upstream side of the reservoir tank 18 between the cooling unit 16 and the reservoir tank 18.

As illustrated in FIG. 8, at the time of preparation for treatment, the first valve 14a is closed and the second valve 14b is opened, and cooling water is short-circuited in the cooling unit 16, the reservoir tank 18, and the pump 20, and does not flow to the circuit cooling block 6. On the other hand, as illustrated in FIG. 9, at the time of treatment, the first valve 14a is opened and the second valve 14b is closed, and the cooling water circulates through the cooling unit 16, the circuit cooling block 6, the reservoir tank 18, and the pump 20. The opening/closing operation of the first valve 14a and the second valve 14b is performed in accordance with an operation on the operation unit 5.

The cooling unit 16 is configured to cool the water circulating to the circuit cooling block 6. The cooling unit 16 includes, for example, a Peltier element. The cooling unit 16 may include a heat exchanger that cools water, and may include, for example, a radiator. Furthermore, the cooling unit 16 may be a refrigerator such as a circulating constant-temperature water tank by a compressor using a chlorofluorocarbon gas as a refrigerant. In this case, the cooling unit 16, the reservoir tank 18, and the pump 20 may be provided in a casing different from the photodynamic therapy device 2 including the circuit cooling block 6 and the irradiation unit 4. Note that in order to suppress freezing, an antifreeze liquid may be used as the cooling water.

In the present embodiment, the cooling unit 16, the first valve 14a and the second valve 14b, the reservoir tank 18, and the pump 20 constituting the cooling water circulation unit are accommodated in the lower casing 8 of the photodynamic therapy device 2 illustrated in FIGS. 1A and 1B.

### 2.2. [Operation of cooling water circulation unit connected to photodynamic therapy device]

Next, an operation of the cooling water circulation unit connected to the photodynamic therapy device according to the embodiment will be described.

Referring again to FIG. 8, the cooling water circulation unit illustrated in FIG. 8 is in a state at the time of preparation for treatment. At the time of preparation for treatment, the first valve 14a is closed and the second valve 14b is opened by the operation on the operation unit 5. The cooling water is short-circuited in the cooling unit 16, the reservoir tank 18, and the pump 20, and does not flow to the circuit cooling block 6.

As described above, during the preparation for treatment, the cooling water is not circulated into the circuit cooling block 6, and is short-circuited in the cooling unit 16, the reservoir tank 18, and the pump 20, whereby the cooling water is cooled to a target temperature.

This has mainly two purposes. One is to avoid sending the cooled saline solution into the patient's body immediately after the start of treatment. That is, before starting the treatment, the blood circuit is initially filled with physiological saline. At this time, when the circuit holder 22 including the blood circuit is installed on the circuit cooling block 6 that has been sufficiently cooled, the temperature of the physiological saline in the blood circuit is further lowered by the circuit cooling block 6 without being raised by the irradiation light. It is required that this lowered temperature saline should be prevented from being fed into the body of the patient.

Another purpose is to efficiently cool the water at the time of preparation for treatment by short-circuiting.

Subsequently, referring to FIG. 9 again, the cooling water circulation unit illustrated in FIG. 9 is in a state at the time of treatment. At the time of treatment, the first valve 14a is opened and the second valve 14b is closed by the operation on the operation unit 5. The cooling water circulates through the cooling unit 16, the circuit cooling block 6, the reservoir tank 18, and the pump 20. As a result, the blood temperature rise caused by the heat generation of the blood due to the absorption of the irradiation light is suppressed.

### 2.3. [Summary]

The photodynamic therapy device 2 according to the present embodiment is a photodynamic therapy device that irradiates light from a light source onto blood that has been taken out of a patient's body and is flowing in the blood tube 34, the blood having absorbed a photoreactive agent, to destroy an undesirable component in the blood or affect the component. The photodynamic therapy device 2 includes the irradiation unit 4 that includes a light source and irradiates the blood in the blood tube 34 with light, and the circuit cooling block 6 that cools the blood in the blood tube 34. The circuit cooling block 6 is connected to the pump 20 that circulates cooling water in the circuit cooling block 6, the reservoir tank 18, and the cooling unit 16 that cools water by the water flow path 12 for flowing the cooling water.

By configuring the photodynamic therapy device 2 in this manner, in the photodynamic therapy, the temperature rise caused by the heat generation of the blood due to the absorption of the irradiation light is suppressed.

### 3. [Other embodiments]

As described above, the embodiment has been described as an example of the technique disclosed in the present application. However, the technique in the present disclosure is not limited thereto, and can also be applied to embodiments in which changes, replacements, additions, omissions, and the like are made as appropriate.

As described above, the temperature of the blood that can be increased by the irradiation unit 4 is required to be suppressed to 40°C or lower. Therefore, for example, in the blood circuit, a temperature sensor that detects the temperature of the blood inside the blood tube 34 immediately before returning to the circulatory organ of the patient in real time may be provided, and a controller that controls the cooling capacity of the cooling unit 16 based on a detection value of the temperature sensor may be further provided. That is, when the temperature sensor detects a temperature close to 40°C (for example, 39°C), the controller that has received the detection value may increase a current and/or voltage flowing through the Peltier element of the cooling unit 16 to further increase the cooling capacity. Furthermore, when the temperature sensor detects a temperature lower than a normal body temperature (for example, 34°C), the controller that has received the detection value may be configured to decrease the current and/or voltage flowing through the Peltier element of the cooling unit 16 to further decrease the cooling capacity.

Furthermore, the accompanying drawings and the detailed description have been provided in order to describe the embodiments. Therefore, the components illustrated in the accompanying drawings and described in the detailed description not only include components essential for solving the problem but also can include, to exemplify the techniques, components that are not essential for solving the problem. For this reason, it should not be immediately recognized that those unnecessary components are necessary only because those unnecessary components are described in the accompanying drawings or the detailed description.

Furthermore, since the above-described embodiments are intended to illustrate the technique in the present disclosure, various changes, replacements, additions, omissions, and the like can be made within the scope of the claims.

### Reference Numerals

- 2: photodynamic therapy device
- 4: irradiation unit
- 6: circuit cooling block
- 6b: pedestal portion
- 6f: fixed circuit cooling sub-block
- 6s: sliding circuit cooling sub-block
- 8: lower casing
- 10: elastic body
- 11: shaft
- 12: water flow path
- 14a: first valve
- 14b: second valve
- 16: cooling unit
- 18: reservoir tank
- 20: pump
- 22: circuit holder
- 24: aluminum sheet metal
- 25: guide
- 26: circuit holder side surface portion
- 27: rib
- 28: circuit holder end portion
- 30: circuit holder center portion
- 32: circuit holder upper surface portion
- 34: blood tube
- 40: tube

## Claims

1. A photodynamic therapy device that irradiates light from a light source onto blood that has been taken out of a patient's body and is flowing in a blood tube (34), the blood having absorbed a photoreactive agent, to destroy an undesirable component in the blood or to affect the component, the photodynamic therapy device (2) comprising:
an irradiation unit (4) that includes the light source and irradiates the blood in the blood tube (34) with light and; and
a circuit cooling block (6) that cools the blood in the blood tube (34),
**characterized in that** the circuit cooling block (6) is connected to a pump (20) that circulates cooling water to the circuit cooling block (6), a reservoir tank (18), and a cooling unit (16) that cools water, by a water flow path (12) that flows the cooling water,
wherein the photodynamic therapy device (2) further comprises a circuit holder (22) including the blood tube (34) and a winding core portion around which the blood tube (34) is disposed and wound,
wherein the circuit cooling block (6) is brought into contact with an inner surface of the circuit holder (22) to cool the blood in the blood tube (34).

2. The photodynamic therapy device according to claim 1, wherein the irradiation unit (4) is configured to be relatively movable with respect to the circuit holder (22) and the circuit cooling block (6).

3. The photodynamic therapy device according to claim 1, further comprising:
a first valve (14a) provided on an upstream side of the circuit cooling block (6) between the cooling unit (16) and the circuit cooling block (6); and
a second valve (14b) provided on an upstream side of the reservoir tank (18) between the cooling unit (16) and the reservoir tank (18),
wherein the photodynamic therapy device (2) is configured to short-circuit water in the cooling unit (16), the reservoir tank (18), and the pump (20) by closing the first valve (14a) and opening the second valve (14b) when preparing for treatment.

4. The photodynamic therapy device according to claim 3, wherein
The cooling unit (16), the circuit cooling block (6), the reservoir tank (18), and the pump (20) are configured to circulate water, during treatment, by opening the first valve (14a) and closing the second valve (14b).

5. The photodynamic therapy device according to claim 1, wherein
the circuit cooling block (6) includes a plurality of circuit cooling sub-blocks (6f, 6s),
one or more elastic bodies (10) are sandwiched between a pair of the circuit cooling sub-blocks (6f, 6s) provided facing each other, and
the circuit cooling block (6) is configured to come into stronger contact with the inner surface of the circuit holder (22) by being biased by the one or more elastic bodies (10).

## Patentansprüche

1. Photodynamische Therapievorrichtung, die Licht aus einer Lichtquelle auf Blut bestrahlt, das aus dem Körper eines Patienten entnommen wurde und in einem Blutschlauch (34) fließt, wobei das Blut einen photoreaktiven Wirkstoff absorbiert hat, um eine unerwünschte Komponente im Blut zu zerstören oder auf die Komponente einzuwirken, wobei die photodynamische Therapievorrichtung (2) Folgendes umfasst:
eine Bestrahlungseinheit (4), die die Lichtquelle einschließt und das Blut in dem Blutschlauch (34) mit Licht bestrahlt und; und
einen Kühlkreislaufblock (6), der das Blut in dem Blutschlauch (34) kühlt,
**dadurch gekennzeichnet, dass** der Kühlkreislaufblock (6) mit einer Pumpe (20) verbunden ist, die Kühlwasser zu dem Kühlkreislaufblock (6), einem Speicherbehälter (18) und einer Kühleinheit (16), die Wasser kühlt, über einen Wasserströmungsweg (12) zirkuliert, durch den das Kühlwasser strömt,
wobei die photodynamische Therapievorrichtung (2) weiter eine Kreislaufhalterung (22) umfasst, die den Blutschlauch (34) und einen Wickelkernabschnitt einschließt, um den der Blutschlauch (34) angeordnet und gewickelt ist,
wobei der Kühlkreislaufblock (6) mit einer Innenfläche der Kreislaufhalterung (22) in Kontakt gebracht wird, um das Blut in dem Blutschlauch (34) zu kühlen.

2. Photodynamische Therapievorrichtung nach Anspruch 1, wobei die Bestrahlungseinheit (4) so konfiguriert ist, dass sie relativ zur Kreislaufhalterung (22) und zum Kühlkreislaufblock (6) beweglich ist.

3. Photodynamische Therapievorrichtung nach Anspruch 1, weiter umfassend:
ein erstes Ventil (14a), das auf einer stromaufwärtigen Seite des Kühlkreislaufblocks (6) zwischen der Kühleinheit (16) und dem Kühlkreislaufblock (6) bereitgestellt ist; und
ein zweites Ventil (14b), das an einer stromaufwärtigen Seite des Speicherbehälters (18) zwischen der Kühleinheit (16) und dem Speicherbehälter (18) bereitgestellt ist,
wobei die photodynamische Therapievorrichtung (2) so konfiguriert ist, dass sie Wasser in der Kühleinheit (16), dem Speicherbehälter (18) und der Pumpe (20) kurzschließt, indem sie das erste Ventil (14a) schließt und das zweite Ventil (14b) öffnet, wenn die Behandlung vorbereitet wird.

4. Photodynamische Therapievorrichtung nach Anspruch 3, wobei
die Kühleinheit (16), der Kühlkreislaufblock (6), der Speicherbehälter (18) und die Pumpe (20) so konfiguriert sind, dass sie während der Behandlung Wasser zirkulieren lassen, indem das erste Ventil (14a) geöffnet und das zweite Ventil (14b) geschlossen wird.

5. Photodynamische Therapievorrichtung nach Anspruch 1, wobei
der Kühlkreislaufblock (6) eine Vielzahl von Kühlkreislauf-Unterblöcken (6f, 6s) einschließt,
ein oder mehrere elastische Körper (10) zwischen einem Paar der einander gegenüberliegend angeordneten Kühlkreislauf-Unterblöcke (6f, 6s) eingeschoben sind, und
der Kühlkreislaufblock (6) so konfiguriert ist, dass er durch die Vorspannung durch den einen oder die mehreren elastischen Körper (10) in stärkeren Kontakt mit der Innenfläche der Kreislaufhalterung (22) kommt.

## Revendications

1. Dispositif de thérapie photodynamique qui irradie de la lumière d'une source lumineuse sur du sang qui a été prélevé du corps d'un patient et qui s'écoule dans un tube (34) de prélèvement sanguin, le sang ayant absorbé un agent photoréactif, pour détruire un composant indésirable dans le sang ou affecter le composant, le dispositif (2) de thérapie photodynamique comprenant :
une unité (4) d'irradiation qui inclut la source lumineuse et irradie le sang dans le tube (34) de prélèvement sanguin avec de la lumière ; et
un bloc (6) de refroidissement de circuit qui refroidit le sang dans le tube (34) de prélèvement sanguin,
**caractérisé en ce que** le bloc (6) de refroidissement de circuit est relié à une pompe (20) qui fait circuler de l'eau de refroidissement vers le bloc (6) de refroidissement de circuit, un réservoir (18), et une unité (16) de refroidissement qui refroidit l'eau, via un trajet (12) d'écoulement d'eau qui fait circuler l'eau de refroidissement,
dans lequel le dispositif (2) de thérapie photodynamique comprend en outre un support (22) de circuit incluant le tube (34) de prélèvement sanguin et une partie de noyau d'enroulement autour de laquelle le tube (34) de prélèvement sanguin est disposé et enroulé,
dans lequel le bloc (6) de refroidissement de circuit est mis en contact avec une surface interne du support (22) de circuit pour refroidir le sang dans le tube (34) de prélèvement sanguin.

2. Dispositif de thérapie photodynamique selon la revendication 1, dans lequel l'unité (4) d'irradiation est configurée pour être relativement mobile par rapport au support (22) de circuit et au bloc (6) de refroidissement de circuit.

3. Dispositif de thérapie photodynamique selon la revendication 1, comprenant en outre :
une première soupape (14a) fournie sur un côté amont du bloc (6) de refroidissement de circuit entre l'unité (16) de refroidissement et le bloc (6) de refroidissement de circuit ; et
une seconde soupape (14b) fournie sur un côté amont du réservoir (18) entre l'unité (16) de refroidissement et le réservoir (18),
dans lequel le dispositif (2) de thérapie photodynamique est configuré pour court-circuiter l'eau dans l'unité (16) de refroidissement, le réservoir (18), et la pompe (20) en fermant la première soupape (14a) et en ouvrant la seconde soupape (14b) lors de la préparation au traitement.

4. Dispositif de thérapie photodynamique selon la revendication 3, dans lequel
l'unité (16) de refroidissement, le bloc (6) de refroidissement de circuit, le réservoir (18), et la pompe (20) sont configurés pour faire circuler l'eau, pendant le traitement, en ouvrant la première soupape (14a) et en fermant la seconde soupape (14b).

5. Dispositif de thérapie photodynamique selon la revendication 1, dans lequel
le bloc (6) de refroidissement de circuit inclut une pluralité de sous-blocs (6f, 6s) de refroidissement de circuit,
un ou plusieurs corps (10) élastiques sont placés entre une paire de sous-blocs (6f, 6s) de refroidissement de circuit disposés face à face ; et
le bloc (6) de refroidissement de circuit est configuré pour entrer en contact accru avec la surface intérieure du support (22) de circuit en étant contraint par les un ou plusieurs corps (10) élastiques.
